Europäisches Patentamt

European Patent Office

Office européen des brevets

(11) Publication number: 0 301 879
A2

# (12) EUROPEAN PATENT APPLICATION

(21) Application number: 88306999.9

(22) Date of filing: 29.07.88

(51) Int. Cl.⁴: **C 07 C 51/50**
C 07 C 51/44, C 07 C 57/04,
C 07 C 67/62, C 07 C 67/54,
C 07 C 69/54

(30) Priority: 31.07.87 US 80526

(43) Date of publication of application:
01.02.89 Bulletin 89/05

(84) Designated Contracting States:
AT BE CH DE ES FR GB GR IT LI LU NL SE

(71) Applicant: HOECHST CELANESE CORPORATION
Route 202-206 North
Somerville, N.J. 08876 (US)

(72) Inventor: Scates, Mark O.
1801 Crooked Creek
Pearland Texas (US)

Dougherty, Edward F.
6224 Creekside Lane
League City Texas (US)

(74) Representative: De Minvielle-Devaux, Ian Benedict Peter
et al
CARPMAELS & RANSFORD 43, Bloomsbury Square
London WC1A 2RA (GB)

(54) Inhibition of polymerization during distillation of monomers.

(57) Unintentional or premature polymerization of ethylenically unsaturated organic compounds during distillation for purification and recovery is inhibited by means of a liquid phase-vapor phase inhibitor system comprising a liquid phase inhibitor containing a phenolic inhibitor and a soluble manganese or cerium compound and a vapor phase inhibitor selected from the group of nitric oxide and N-phenyl-N-nitrosohydroxylamine ammonium salt.

EP 0 301 879 A2

Bundesdruckerei Berlin

Description

## INHIBITION OF POLYMERIZATION DURING DISTILLATION OF MONOMERS

### BACKGROUND OF THE INVENTION

#### 1. Field of the Invention

The present invention relates to a method for inhibiting unintentional or premature polymerization of ethylenically unsaturated organic compounds, such as acrylic acid and monomeric acrylates, during the distillation thereof, and more particularly to the distillation of ethylenically unsaturated organic compounds in the presence of liquid phase inhibitors in combination with vapor phase inhibitors.

#### 2. Description of the Prior Art

Ethylenically unsaturated organic compounds, such as acrylic acid, methacrylic acid, methacrylic esters, acrylate esters, and the like, are widely used in the production of homopolymers and copolymers. These homopolymers and copolymers, produced readily through the polymerization of the available double bonds of the organic compounds, are widely used in paints, coatings, lacquers and the like. The olefinic activity of the ethylenically unsaturated organic compounds, makes the polymerized products highly useful for many purposes. On the other hand, the olefinic activity poses a problem of unintentional or premature polymerization promoted by light and heat. This tendency is exhibited on storage, shipping and during purification of these polymerizable unsaturated compounds where distillation at higher temperatures are involved. During distillation the liquid phase in the distillation unit should contain a liquid phase nonvolatile inhibitor and the column and condenser should contain a volatile inhibitor such as nitric oxide to prevent the undesired or premature polymerization of the ethylenically unsaturated organic monomer product.

U. S. 2,741,583 to Vaughn et al discloses the use of alkali metal nitrites, such as sodium nitrite, or a mixture of nitrogen oxides as obtained by the action of an acid on such a nitrite, as polymerization inhibitors during the distillation of methyl or ethyl acrylates. British Patent GB 1,265,419 discloses a method for minimizing polymerization of acrylic acid during distillation thereof by distilling the acrylic acid in the presence of nitric oxide in the gas phase and phenothiazine in the liquid phase.

It is also known to use a metal additive in combination with phenolic inhibitors to prevent undesirable polymerization of ethylenically unsaturated organic compounds. In Chemical Abstracts, Vol. 74, 1971, 112642V, a Japanese patent (No. 70-35,285) filed June 1, 1967, assigned to Nippon Kayaku Co., Ltd., describes the mixture of chromium acetate with hydroquinone as a satisfactory inhibitor for acrylic and methacrylic acid. In Japanese patent publication No. 51-98211, filed February 19, 1975, assigned to Sumitomo Chemical Co., Ltd., the combination of manganese salt and phenolic type inhibitors provide satisfactory stabilization of acrylic acid. U. S. Patents 4,507,495 and 4,542,231 disclose the use of cerium and manganese compounds together with phenolic type inhibitors for stabilization of ethylenically unsaturated organic compounds, such as ethyl acrylate, against unintentional polymerization. In U. S. 4,542,231, nitric oxide is further disclosed to prevent undesired or premature polymerization of ethylenically unsaturated organic monomer products such as acrylic acid. U. S. 4,210,493 discloses the use of aliphatic and aromatic C- nitroso compounds, such as t-nitrosobutane and nitrosobenzene, to inhibit polymerization of acrylic and methacrylic acids during their preparation, purification and storage. In Portugese Patent No. 78759, dated January 31, 1985, acrylic acid is purified of carbonylic impurities by treatment with an aqueous solution of a hydrazine compound having the formula R R$_1$-N-N-H$_2$ wherein R and R$_1$ are hydrogen, alkyl or alkyl aromatic groups. This patent also discloses the use of standard inhibitors such as amines, hydroquinones, quinone, nitro, nitroso aromatics, N-nitroso-hydroxylamines and mixtures thereof. In U. S. Patent 4,310,676, acrylic acid esters are stabilized against premature polymerization by the addition of phenothiazine and para-nitrosophenol.

### SUMMARY OF THE INVENTION

The present invention is directed to an improvement in the distillation of readily polymerizable ethylenically unsaturated organic compounds such as acrylic acid and ethyl acrylate, by subjecting such compounds to distillation in the presence of a liquid phase-vapor inhibitor system comprising a liquid phase inhibitor containing a phenolic type inhibitor and a soluble manganese or cerium compound in combination with a vapor phase inhibitor containing nitric oxide or N-phenyl-N-nitrosohydroxylamine ammonium salt.

### DESCRIPTION OF THE INVENTION

In a preferred aspect of the invention, inhibition of polymerization may be applied to the distillation of acrylic acid ester obtained by alcohol esterification of acrylic acid, acrylic acid obtained by the catalytic oxidation of propylene, or the distillation of acrylic acid and ethyl acrylate as produced from the interaction of acrylic acid with ethylene in the presence of a sulfuric acid catalyst. As examples of the latter process, see U. S. Patent No. 3,703,539, issued November 21, 1972, to DiLiddo; U. S. Patent No. 3,539,621, issued November 10, 1970, to Cipollone et al; U. S. Patent No. 3,894,076, issued July 8, 1975, to Van Duyne et al; and U. S. Patent No. 4,490,553, issued December 25, 1984, to Chase et al. In these processes, the reaction is believed to involve the formation of intermediate sulfates from the reaction of ethylene with sulfuric acid in a reactor tower wherein the sulfates further react with acrylic acid to form ethyl acrylate. To provide a product mixture in good overall yields with high carbon efficiencies, the reaction mixture is sent from the reaction tower to a distillation train where the mixture is distilled to obtain liquid ethyl acrylate with unreacted ethylene, acrylic acid and sulfuric acid being recycled to the reactor. During distillation of the acrylate monomer, polymer formation and fouling occur due to the polymerization of unihibited condensing vapor. Although it would be expected that polymer fouling would be inhibited by commonly used liquid phase inhibitors such as phenothiazine, hydroquinone, p-methyloxyphenols and the like, the distillation of acrylate monomers is exacerbated by the polymerization of uninhibited condensing vapor (vapor phase polymerization) which does not contain a liquid phase inhibitor.

Reaction products withdrawn from the reactor tower are sent to a purification train comprising a recovery distillation tower, light ends distillation tower and finishing tower, all of which are of conventional design. In the recovery distillation tower, products withdrawn from the reactor tower are introduced through a pressure reduction valve and maintained under vacuum by conventional means so that the pressure is less than about 500 nm of mercury absolute. The still pot temperature is maintained within the range of about 100°C to 170°C, preferably 100°C to 130°C, and the still head temperature within the range of about 28°C to 45°C, preferably about 30°C to 40°C. A vacuum is maintained in the recovery column at reduced pressures less than atmospheric so that the pressure is within the range of 300 to 450 mm of mercury absolute. The distillation section of the recovery distillation tower is of conventional design and may contain packing, sieve type trays or dual flow trays. The distillation section will contain an equivalent of at least four theoretical trays. The residence time of the reaction products in the base of the distillation tower should be as low as possible because at temperatures required in the reboiler for vaporization some polymerization may occur. It is desirable to have a feed stream lean in acrylic acid being fed to the recovery distillation tower since this will result in less polymer formation.

Light ends of crude ethyl acrylate, comprising mainly ethyl acrylate, small amounts of unreacted ethylene and other uncondensables, are removed overhead from the recovery distillation tower and passed to a light ends distillation tower of conventional distillation design. A stream comprising mainly unreacted ethylene is removed as overhead from the light ends distillation tower and may be disposed of or recycled to the reactor tower as desired. The bottoms product from the light ends distillation tower is a partially purified ethyl acrylate product which is passed to a finishing distillation tower where a substantially pure ethyl acrylate product is recovered by fractionation. The ethyl acrylate residue product from the finishing distillation tower is removed as a bottoms product and can be recycled to the reactor tower or a portion thereof sent to a wiped-film evaporator for recovery of organic products such as ethyl acrylate and acrylic acid.

The cerium or manganese compound used as liquid phase inhibitors in accordance with the invention can be any compound which is soluble in the polymerizable ethylenically unsaturated compound, e.g., acrylic acid, methacrylic acid, ethyl acrylate, etc., or the product mixtures resulting from their preparation. Suitable manganese compounds include, among others, manganous acetate, manganese naphthanate, manganese carbonate, manganese octoate, manganous nitrite, manganous propionate, manganous nitrate, manganous oxide, manganous hydroxide, manganous chloride, manganous phosphate, manganous perchlorate and the like. Suitable cerium compounds include, among others, ceric ammonium nitrate, cerous acetate, cerous ammonium sulfate, cerium carbonate, cerium nitrate, cerium naphthanate, cerous benzoate, cerous nitrate, cerium octoate, cerous oxalate and the like. The amount employed will range from about 5 parts per million to about 5,000 parts per million, preferably from about 25 to about 500 parts per million of the total reaction mixture or individual product.

The vapor phase inhibitors which are employed in accordance with the invention are nitric oxide (NO) or N-phenyl-N-nitrosohydroxylamine ammonium salt (NPH). In the processing of acrylic acid, ethyl acrylate and other polymerizable ethylenically unsaturated organic compounds, the combined presence of these liquid and vapor phase inhibitors permits the use of increased temperatures and pressures, thereby increasing the capacity of distillation columns for purification and recovery. The amount of inhibitor employed will range from 5 parts per million to 5,000 parts per million but in most cases, for economic reasons, the inhibitor will be used in amounts ranging from 20 to 1,000 parts per million based on the product or product mixture.

The phenolic inhibitors which are used herein include known inhibitors of the phenolic type such as dihydroxybenzene derivatives such as hydroquinone, catechol, resorcinol, dihydroxyxylene, methoxyphenols such as guaiacol and p-methoxyphenol (methyl ether of hydroquinone), pyrogallol, methylpyrogallol, cresols, phenol, xylenols, 4,4-thiobis-6-tertiarybutyl-3-methylphenol and the like. The amounts of phenol type inhibitors used in this invention are from about 5 parts per million to about 5,000 parts per million, preferably from about 25 parts per million to about 500 parts per million based on the product or total reaction product mixture.

A wide variety of ethylenically unsaturated organic compounds may be inhibited against unintentional or premature polymerization. Such compounds include acrylic acid, methacrylic acid, vinyl acetate, styrene, acrylonitrile, vinyl chloride, acrylamide, N-methylolacrylamide, glycidyl methacrylate, and the like; an alkyl acrylate ester wherein the alkyl group contains from 1 to 10 carbon atoms such as methyl acrylate, ethyl acrylate, n-butyl acrylate, octyl acrylate, isoctyl acrylate, 2-ethylhexyl acrylate and the like; an alkyl methacrylate ester wherein the alkyl group contains from 1 to 10 carbon atoms such as methyl methacrylate, ethyl methylacrylate, butyl methacrylate, and the like; an acrylate ester prepared by the reaction of acrylic acid and a saturated aliphatic polyol having a 2 to 10 carbon atoms and 2 to 5 hydroxy groups, said ester containing 2 to 5 acrylate groups such as pentaerythritol triacrylate, trimethyl propane triacrylate, 1,6-hexandiol diacrylate, tetraethylene glycol diacrylate, tripropylene glycol diacrylate and trimethylol propane trimethacrylate and the like.

This invention will be further described in the following examples:

## EXAMPLE 1

One liter of acrylic acid (HAca) containing 500 ppm manganese ion (as the acetate) and 200 ppm of MeHQ was charged to a flask heated to 103°C under a vacuum of 150 mm Hg absolute. After 300 ml of HAca was distilled overhead, it was observed that a white polymer residue had accumulated in the flask and overhead condensor.

The above experiment was repeated using 200 ppm MeHQ, 20 ppm manganese (as the acetate) and 20 ppm NPH (N-phenyl-N-nitrosohydroxylamine ammonium salt). No polymer developed during this run.

## EXAMPLE 2

800 ml of HAca containing 200 ppm manganese ion (as the acetate) plus 200 ppm MEHQ was charged to a flask heated to 103°C under a vacuum of 150 mm Hg absolute. A 0.1 wt.% aqueous solution (1000 ppm) of NPH (N-phenyl-N-nitrosohydroxylamine ammonium salt) was continuously fed with 10 ppm manganous acetate solution to the flask. The flask was purged with nitrogen and 400 ml of HAca was thereafter distilled overhead. No polymer residue appeared anywhere in the flask column or overhead condenser. Previous test in the absence of the NPH component developed considerable polymer in the overhead condenser.

## EXAMPLE 3

800 ml of Acrylic Acid containing 10 ppm manganese ion (as the acetate) and 200 ppm MeHQ (hydroquinone monomethyl ether) were charged to a flask heated to 102°C under a vacuum of 150 mm Hg absolute. A 0.1 wt.% aqueous solution of (1000 ppm) of NPH (N-nitroso-N-phenylhydroxylamine ammonium salt) was continuously fed to the flask. The flask was purged with nitrogen for 30 minutes and 400 ml of acrylic acid was thereafter distilled overhead in 3-1/2 hours. No polymer residue appeared in the flask, column or overhead condenser. Previous tests in the absence of the Mn component developed a polymeric film on the walls of the base flask.

## EXAMPLE 4

100 ml of HAca containing 10 ppm manganese ion (as the acetate) and 200 ppm MeHQ (hydroquinone monomethyl ester) were charged to a flask heated to 145°C and refluxed at one atomosphere of pressure. A 1000 ppm aqueous solution of NPH was continuously fed to the flask at 1 ml solution/30 min. No polymer was observed anywhere in the system after 2 hours of refluxing.

A. The same experiment was repeated by sparging NO in the base instead of adding NPH. Only a slight turbidity was observed to form in the base liquid after 2 hours of refluxing.

B. The same experiment was repeated again in the absence of NPH and NO (no vapor phase component). The base liquid remained inhibited by the MEHQ/Mn. However, the base liquid accumulated polymer which had formed in the reflux condenser and dropped into the base liquid. The reflux condenser plugged with the polymer after one hour (inadequate inhibition of condensing vapor).

C. The same experiment was repeated using only 200 ppm MEHQ and a slow NO sparge (no Mn present). The base solution formed a thick film of polymer on the flask wall and eventually became one solid mass of polymer. This indicated inadequate liquid phase inhibition. No polymer was found in the reflux condenser.

## Claims

1. A method for preparing a polymerizable ethylenically unsaturated organic compound wherein a reaction product or product mixture is subject to distillation for purification and recovery, wherein the distilling of said product or product mixture is carried out in the presence of a liquid phase-vapor phase inhibitor system comprising a liquid phase inhibitor containing a phenolic inhibitor and a soluble manganese or cerium compound in combination with a vapor phase inhibitor selected from nitric oxide and N-phenyl-N-nitrosohydroxylamine ammonium salt.

2. The method of claim 1 wherein the cerium or manganese compound is present in an amount ranging from 5 parts per million to 5,000 parts per million and the nitric oxide or N-phenyl-N-nitrosohydroxylamine ammonium salt is present in an amount ranging from 5 parts per million to 1000 parts per million.

3. The method of claim 1 or 2 wherein the phenolic type inhibitor is added to the reaction product or product mixture in an amount ranging from 5 parts per million to 5,000 parts per million.

4. The method of any claims 1-3 wherein the phenolic inhibitor is selected from hydroquinone and the monomethyl ether of hydroquinone, catechol, guaiacol, and resorcinol.

5. The method of any of claims 1-4 wherein the cerium compound is selected from cerous acetate, ceric ammonium nitrate, cerium naphthanate, cerium octoate and cerium carbonate and the manganese compound is selected from manganous acetate, manganese naphthanate, manganese octoate, manganese oxide and manganese carbonate.

6. The method of any of claims 1-5 wherein the polymerizable ethylenically unsaturated compound is selected from acrylic acid, methacrylic acid, methyl acrylate ethyl acrylate, butyl acrylate and 2-ethylhexyl acrylate.

7. The method of any of claims 1-6 wherein the distillation is carried out under vacuum at pressures less than 500 mm Hg absolute at temperatures in the range of 50°C to 170°C.